# EUROPEAN PATENT APPLICATION

(11) **EP 2 759 289 A1**
(43) Date of publication of application: **30.07.2014**
(21) Application number: 13000359.3
(22) Date of filing: 24.01.2013
(51) Int. Cl.: A61K 6/087, C07D 403/06, C07D 403/12, C07D 405/14, C07D 487/04

(54) **Polymerizable dental composition**

(71) Applicant: DENTSPLY DETREY GmbH, 78467 Konstanz (DE)
(72) Inventor: E, Klee, Joachim, 78315 Radolfzell (DE); Retzmann, Nils, 40215 Düsseldorf (DE); Ritter, Helmut, 42111 Wuppertal (DE); Szillat, Florian, 40474 Düsseldorf (DE)
(74) Representative: Hartz, Nikolai

(57) **Abstract**

A dental composition comprising a compound of the following formula (I) 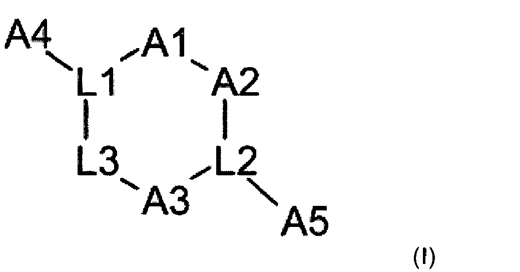 wherein A1, A2, A3, A4, and A5 are as defined in claim 1.

## Description

### Field of the invention

The present invention relates to a polymerizable dental composition comprising a novel polymerizable imidic and/or polyamic compound. Moreover, the present invention relates to a process for the preparation of a dental composition comprising a novel polymerizable imidic and/or polyamic compound, a composition obtainable by the process of the present invention and the use of the novel compound for the preparation of a dental composition. The composition of the present invention comprising the novel polymerizable imidic and/or polyamic compound is useful and safe for dental applications and has low shrinkage upon polymerization and does not pose a health risk due to release of potentially hazardous degradation products.

### Background of the invention

Polymerizable dental compositions comprise of an organic matrix containing polymerizable monomers. A high molecular weight monomer is preferred as component of the organic matrix because low molecular weight monomers such as methyl methacrylate lead to high shrinkage upon curing and increased stress at the interface of restoration. Presently, as a polymerizable high molecular weight monomer, Bis-GMA (bisphenol A-glycidyl methacrylate) (US Patent 3,179,623) is most commonly used.

However, in recent years health risks due to the release of unreacted monomers and potentially hazardous degradation products of components of dental composites became a matter of widespread concerns about the safety of use of dental composites in specific populations. Dental composites based on Bis-GMA, in particular, were found to increase estrogenic activity in treated subjects and the use of resins containing Bis-GMA appears to contribute to human exposure to xenoestrogens, particularly in children and adolescents (Olea N. et al., Environ Health Perspect. 1996 March; 104(3): 298-305). This is generally attributed to the release of bisphenol A upon degradation of compounds like Bis-GMA.

Bisphenol A is an endocrine disruptor, which can mimic the body's hormones such as estrogen and is reported to have serious adverse effects on patient health in various ways (vom Saal F. S. and Myers J. P., JAMA 2008 September ; 300(11): 1353-1355). Recently, bisphenol A has even been officially declared a "toxic substance" by Canadian regulatory agencies in September 2010.

Therefore, a dental composition having favorable properties for the use in dentistry but' which is not based on a bisphenol A-containing monomer is desirable.

EP 1 046 657 discloses photocurable resin compositions containing imidated acrylic compounds.

### General description of the invention

It is an object of the present invention to provide a dental composition which has favorable viscosity, reduced polymerization shrinkage, high biocompatibility and a refractive index which is well-adapted to the glass filler in order to provide low-opacity polymerizable dental compositions allowing photopolymerization of the dental composition to a sufficient depth. Specifically, it is an object to provide a dental composition providing low shrinkage upon curing and which does not expose the patient to substances which could be detrimental to the patient's health, such as xenoestrogens. Furthermore, it is an object of the present invention to provide a process for the preparation of said dental composition.

The present invention provides a dental composition comprising a compound of the following formula (I) 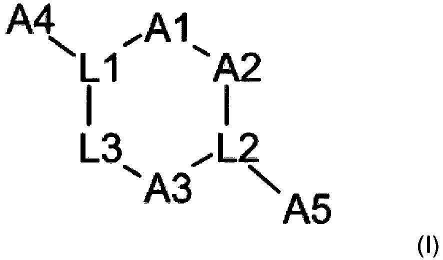 wherein A1, A2, A3, A4, and A5 independently represents a group, which is either present or absent, provided that at least two groups selected from A1, A2, A3, A4, and A5 are present, each group A1, A2, A3, A4, and A5, when present, independently representing a moiety comprising
■ a linear, branched or cyclic hydrocarbon fragment having 0 to 18 carbon atoms and optionally 1 to 5 groups selected from the group of -O-, -NH- and -S- atoms and which may be substituted by 1 to 5 functional groups selected from the group of carboxylic acid groups or hydroxyl groups; and
■ a fragment selected from the following fragments (a) to (c): 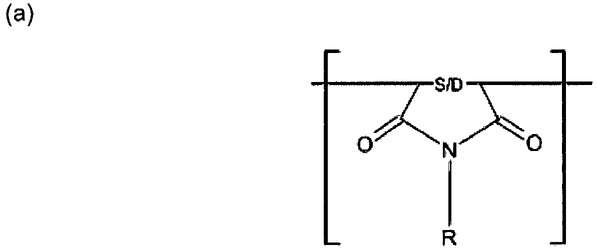 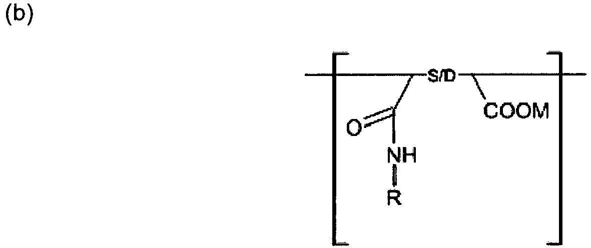 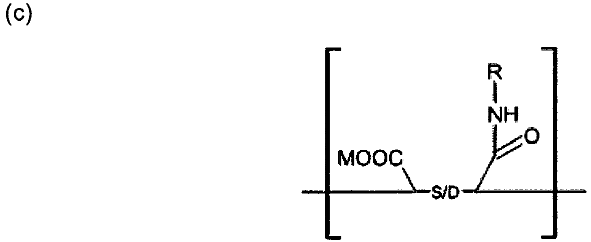 wherein
   M represents a hydrogen atom or a monovalent cation;
   S/D represents a single bond or a double bond whereby the double bond may be part of an aromatic ring;
   R represents a groups L(X)ₙ,
   wherein
   L represents a single bond or a linear, branched or cyclic (n+1) valent hydrocarbon group having 2 to 18 carbon atoms which may contain 1 to 5 heteroatoms selected from the group of oxygen, nitrogen and sulfur atoms, and which may be substituted by 1 to 5 functional groups selected from the group of carboxylic acid groups or hydroxyl groups;
   X independently represents either
   - a polymerizable group selected from a (meth)acryloyloxy group, an allyl oxy group, allylamino, vinyloxy, vinylaryl and vinylamino group, or
   - a glycidyl ether, a glycidyl amine, or a glycidyl amide group, whereby in case L is a single bond, a glycidyl moiety forms a glycidyl imide in a fragment of formula (a) and a glycidyl amide in a fragment of any one of formula (b) and (c);
   n is an integer of 1 to 3;
   L1, L2, and L3 are either present or absent, provided that at least one group selected from L1, L2, and L3 is present, and if present, independently represents
• when L1, L2, and L3 is divalent:
   a single bond, a group -O-, -S-, SO₂, or -NR'- (wherein R' represents a hydrogen atom or an alkyl group having 1 to 18 carbon atoms), or a divalent hydrocarbon group having 1 to 8 carbon atoms optionally containing 1 to 5 groups selected from the group of -O-, -NH- and -S- atoms and which may be substituted by 1 to 5 functional groups selected from the group of carboxylic acid groups, and hydroxyl groups; and
• when L1 and L2 is trivalent:
   a group >N- or a trivalent hydrocarbon group having 1 to 8 carbon atoms optionally containing 1 to 5 heteroatoms selected from the group of oxygen, nitrogen and sulfur atoms and which may be substituted by 1 to 5 functional groups selected from the group of carboxylic acid groups and hydroxyl groups;
   said dental composition further comprising a solvent and/or a particulate filler.

Moreover, the present invention provides a process for the preparation of a dental composition comprising a compound of formula (I), which comprises
(i) reacting a compound of the following formula (X) 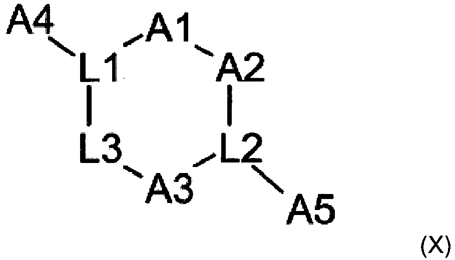 wherein
   A1, A2, A3, A4, and A5 independently represents a group, which is either present or absent, provided that at least two groups selected from A1, A2, A3, A4, and A5 are present, each group A1, A2, A3, A4, and A5, when present, independently representing a moiety comprising
   ■ a linear, branched or cyclic hydrocarbon fragment having 0 to 18 carbon atoms and optionally 1 to 5 groups selected from the group of -O-, -NH- and -S- atoms and which may be substituted by 1 to 5 functional groups selected from the group of carboxylic acid groups and hydroxyl groups; and
   ■ a fragment selected from the following fragments (d): (d) 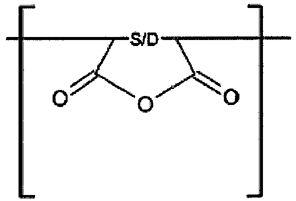
   wherein
   S/D represents a single bond or a double bond whereby the double bond may be part of an aromatic ring; L1, L2, and L3 are either present or absent, provided that at least one group selected from L1, L2, and L3 is present, and if present, independently represents
   ○ when any of L1, L2, and L3 is divalent:
      a single bond, a group -O-, -S-, SO₂, or -NR'- (wherein R' represents a hydrogen atom or an alkyl group having 1 to 18 carbon atoms), or a divalent hydrocarbon group having 1 to 8 carbon atoms optionally containing 1 to 5 groups selected from the group of -O-, - NH- and -S- atoms and which may be substituted by 1 to 5 functional groups selected from the group of carboxylic acid groups and hydroxyl groups; and
   ○ when any of L1 and L2 is trivalent:
      a group >N- or a trivalent hydrocarbon group having 1 to 8 carbon atoms optionally containing 1 to 5 heteroatoms selected from the group of oxygen, nitrogen and sulfur atoms and which may be substituted by 1 to 5 functional groups selected from the group of carboxylic acid groups and hydroxyl groups
   with a compound of the following formula (XI)

   H₂N-L(Y')ₙ (XI)

   wherein L represents a linear, branched or cyclic (n+1) valent hydrocarbon group having 2 to 18 carbon atoms which may contain 1 to 5 heteroatoms selected from the group of oxygen, nitrogen and sulfur atoms, and which may be substituted by 1 to 5 functional groups selected from the group of carboxylic acid groups, and hydroxyl groups; Y' represents a hydroxyl group or an amino group; and n is an integer of 1 to 3; or
   with a compound of the following formula (XII) 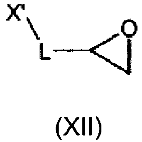 wherein
   L methylene group; and
   X' is an amino group; and
(ii) subsequent to the reaction with a compound of formula (XI), reacting the reaction product with one or more compounds of formula (XII) wherein X' is a leaving group, preferably a halogen atom, or with (meth)acrylic anhydrides for obtaining a compound of formula (I), and further comprising
(iii) combining the compound of formula (I) with a solvent and/or a particulate filler.

Moreover, the present invention provides a dental composition comprising a compound obtainable by the process as defined above.

Moreover, the present invention provides the use of a compound obtainable by the process as defined above for the preparation of a dental composition

Due to the high molecular mass of the polymerizable monomeric compound described herein, the dental composition of the present invention has the advantages of favorable viscosity and small polymerization shrinkage. Furthermore, the inventive composition has high biocompatibility because it is not based on a bisphenol A-containing monomer, thus allowing the use in patients without any problems.

### Description of the preferred embodiments

The compound which is comprised in the dental composition of the present invention is defined by formula (I) representing a simple graph of eight vertices (A1, A2, A3, A4, A5, L1, L2 and L3) which are connected by edges, as shown in formula (I) or formula (X).

The definition of a compound of the invention is based on a representation of objects A1, A2, A3, A4, A5, L1, L2 and L3 representing chemical groups or moieties wherein certain pairs of objects are interconnected by bonds. It is essential that in a compound of formula (I) at least two fragments selected from imide fragments (a) and amic acid fragments (b) and (c) are present as well as at least one polymerizable group Z. The essential moieties are linked in a compound of formula (I) by a specific hydrocarbon group which imparts a low melting point to the compound of formula (I) so that the compound of formula (I) is preferably liquid at room temperature. Preferably, the compound of the present invention is soluble at 25 °C in at least 1.0 % wt/v, more preferably, at least 5 % wt/v in a reactive diluent selected from the group consisting of ethylene glycol dimethacrylate, diethylene glycol dimethacrylate, triethylene glycol dimethacrylate, glycerine dimethacrylate, trimethylolpropane tri(meth)acrylate and 2-hydroxyethyl methacrylate.

A1, A2, A3, A4, A5, L1, L2 and L3 in formula (I) or (X) independently represent a group as further defined below. A1, A2, A3, A4, A5, L1, L2 and L3 in formula (I) or (X) are either present or absent, provided that at least two groups selected from A1, A2, A3, A4 and A5 are present. If two neighboring groups in formula (I) or (X) independently represent a group as defined below, and are present in formula (I) or (X), then it should be understood that these neighboring groups are linked to each other by a chemical bond.

In one preferred embodiment, if any of the groups A1, A2, A3, L1, L2 or L3 is absent, the respective neighboring groups in formula (I) or (X) form a bond with each other. In another preferred embodiment, if any of the groups A1, A2, A3, L1, L2 or L3 is absent, the respective neighboring groups do not form a bond with each other.

Next, the preferred embodiments of a compound represented by formula (I) will be further described.

A1, A2, A3, A4 and A5 independently represent a moiety comprising a fragment selected from the fragments (a) to (c). The fragments (a) to (c) may be combined with a linear, branched or cyclic hydrocarbon fragment (1 to 18 carbon atoms).

In case fragments (a) to (c) are not combined with a linear, branched or cyclic hydrocarbon fragment (0 carbon atoms), fragment (a) to (c) is a monovalent fragment wherein a first valency is directly bonded to any of A1, A2, A3, A4, A5, L1, L2 and L3 and the second valency is linked to a hydrogen atom. Accordingly, fragments (a) to (c) are fragments of the following formula (a') to (c') wherein S/D, R and M are as defined above:

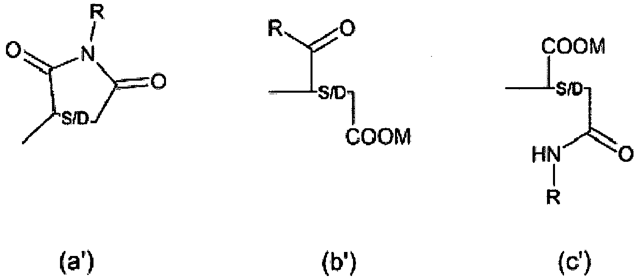

In case fragments (a) to (c) are combined with a linear, branched or cyclic hydrocarbon fragment (1 to 18 carbon atoms), the linear, branched or cyclic hydrocarbon fragment may form a bridge between fragment (a) to (c) and any of A1, A2, A3, A4, A5, L1, L2 and L3.

Accordingly, the linear, branched or cyclic hydrocarbon fragment may be a bridge between fragment (a') to (c') and any of A1, A2, A3, A4, A5, L1, L2 and L3 according to the following formulae (a") to (c"):

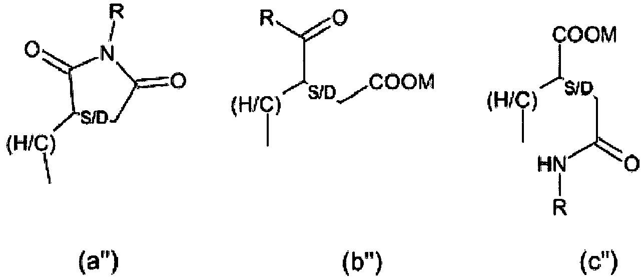

In case the linear, branched or cyclic hydrocarbon fragment forms a bridge between fragment (a) to (c) and any of A1, A2, A3, A4, A5, L1, L2 and L3 according to the above formulae (a") to (c"), the linear, branched or cyclic hydrocarbon fragment may have up to 18 carbon atoms. Preferably, the linear, branched or cyclic hydrocarbon fragment is a saturated alicyclic group having 1 to 8 carbon atoms, more preferably an alkylene group having 1 to 8 carbon atoms.

The hydrocarbon fragment may be form a cyclic structure together with any of fragment (a) to (c) and and be linked to any of A1, A2, A3, A4, A5, L1, L2 and L3 according to the following formulae (a"') to (c"')

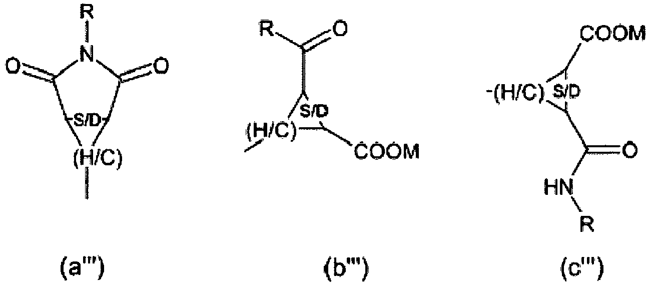

In case the linear, branched or cyclic hydrocarbon fragment forms a cyclic structure according to the above formulae (a"') to (c"'), the linear, branched or cyclic hydrocarbon fragment may have up to 18 carbon atoms. Preferably, the linear, branched or cyclic hydrocarbon fragment contains a saturated alicyclic group or forms an aromatic ring. Preferably, a saturated alicyclic group or an aromatic ring is substituted with one or more linear alkyl groups having 4 to 12 carbon atoms in order to lower the melting point of the compound of formula (I).

According to a preferred embodiment, the cyclic structure comprises an aromatic ring system. The aromatic ring system may be a monocyclic ring system such as a phenyl group or a polycyclic ring system such as a naphthyl group.

The linear, branched or cyclic hydrocarbon fragment may have optionally 1 to 5 groups selected from the group of -O-, -NH- and -S- atoms. Furthermore, the linear, branched or cyclic hydrocarbon fragment may be substituted by 1 to 5 functional groups selected from the group of carboxylic acid groups and hydroxyl groups.

According to a preferred embodiment, the hydrocarbon fragment is a branched hydrocarbon fragment which includes a linear alkyl group having 4 to 12 carbon atoms such as n- butyl, n-pentyl, n-hexyl, n-heptyl, n-octyl, n-nonyl, n-decyl, n-undecyl, or n-dodecyl, whereby n-pentyl, n-hexyl, and n-heptyl are preferred. A branched hydrocarbon fragment which includes a linear alkyl group having 4 to 12 carbon atoms lowers the melting point of the compound of formula (I) which is preferable for a dental composition.

In one embodiment, each group A1, A2, A3, A4 and A5, if present, independently represents a moiety comprising a linear, branched or cyclic hydrocarbon fragment having 0 to 18 carbon atoms and optionally 1 to 5 groups selected from the group of -O-, -NH- and - S- atoms and which may be substituted by 1 to 5 functional groups selected from the group of carboxylic acid groups and hydroxyl groups, preferably a branched or cyclic hydrocarbon fragment having 0 to 3 carbon atoms, and includes a linear alkyl group having 4 to 12 carbon atoms such as n- butyl, n-pentyl, n-hexyl, n-heptyl, n-octyl, n-nonyl, n-decyl, n-undecyl, or n-dodecyl, whereby n-pentyl, n-hexyl, and optionally 1 to 3 groups selected from the group of -O-, -NH- and -S- atoms , and a fragment selected from the following fragments (a) to (c): 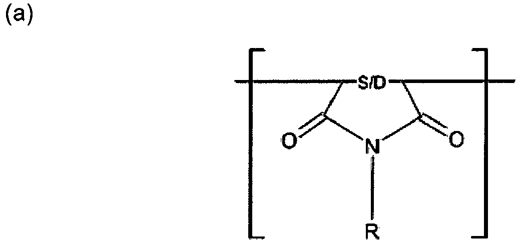 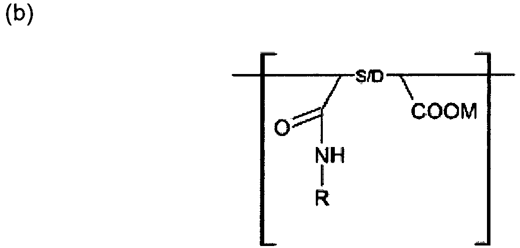 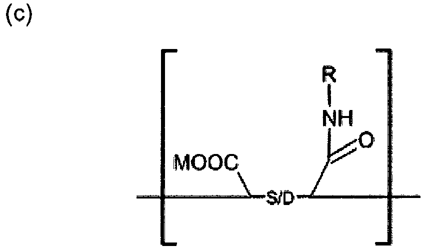 wherein M represents a hydrogen atom or a monovalent cation; S/D represents a single bond or a double bond whereby the double bond may be part of an aromatic ring; R represents a group L(X)ₙ, wherein L represents a single bond or an linear, branched or cyclic (n+1) valent hydrocarbon group having 2 to 18 carbon atoms which may contain 1 to 5 heteroatoms selected from the group of oxygen, nitrogen and sulfur atoms, and which may be substituted by 1 to 5 functional groups selected from the group of carboxylic acid groups and hydroxyl groups; X independently represents either a polymerizable group selected from a (meth)acryloyloxy group, an allyl oxy group, allylamino, vinyloxy, vinylaryl and vinylamino group, or a glycidyl ether, a glycidyl amine, a glycidyl amide group, whereby in case L is a single bond, a glycidyl moiety forms a glycidyl imide in a fragment of formula (a) and a glycidyl amide in a fragment of any one of formula (b) and (c); and n is an integer of 1 to 3.

A monovalent cation may be selected from pharmaceutically acceptable cations such a, lithium, sodium, potassium, ammonium or a quaternaty ammonium cation.

S/D preferably represents a single bond and in case S/D represents a double bond, the double bond forms part of an aromatic ring.

A linear, branched or cyclic (n+1) valent hydrocarbon group having 2 to 18 carbon atoms may preferably be a saturated alicyclic (n+1) valent hydrocarbon group having 2 to 18, more preferably 4 to 12 carbon atoms. A linear, branched or cyclic (n+1) valent hydrocarbon group having 2 to 18 carbon atoms may contain 1 to 5 heteroatoms selected from the group of oxygen, nitrogen and sulfur atoms. The heteroatoms may be present in the carbon chain, thereby being linked to at least two carbon atoms, or be bonded to a single carbon atom as in the case of a carbonyl group.

According to a preferred embodiment, the (n+1) valent hydrocarbon group is a linear alkylene group having 4 to 18, more preferably 6 to 12 carbon atoms.

According to a further preferred embodiment, in case the the (n+1) valent hydrocarbon group is a branched or cyclic hydrocarbon group, the branched or cyclic hydrocarbon group is a saturated alicyclic group which includes as a substituent a linear alkyl group having 4 to 12 carbon atoms such as n- butyl, n-pentyl, n-hexyl, n-heptyl, n-octyl, n-nonyl, n-decyl, n-undecyl, or n-dodecyl, whereby n-pentyl, n-hexyl, and n-heptyl are preferred.

A branched (n+1) valent alicyclic group which includes as a substituent a linear alkyl group having 4 to 12 carbon atoms lowers the melting point of the compound of formula (I) which is preferable in case the dental composition does not contain a solvent.

The (n+1) valent hydrocarbon and which may be substituted by 1 to 5 functional groups selected from the group of carboxylic acid groups and hydroxyl groups.

In a preferred embodiment, the fragment comprised in a group A1, A2, A3, A4 and A5, is fragment (a) shown above. In a specific embodiment, R is a glycidyl group forming a glycidyl imide with fragment (a) comprised in a group A1, A2, A3, A4 and A5.

In another preferred embodiment, S/D in any of fragments (a), (b) or (c) represents a double bond whereby the double bond is part of an aromatic ring.

In another preferred embodiment, n is 1.

In another preferred embodiment, each group A1, A2, A3, A4 and A5, if present, independently represents a fragment (a) as shown above, wherein S/D represents a double bond whereby the double bond is part of an aromatic ring. R represents a group L(X)ₙ, wherein L represents a straight or branched alkylene group having 2 to 18 carbon atoms which may contain 1 to 5 groups selected from the group of -O-, -NH- and -S- atoms, and which may be substituted by 1 to 5 functional groups selected from the group of carboxylic acid groups and hydroxyl groups.

X independently represents either a polymerizable group selected from a (meth)acryloyloxy group, an allyl oxy group, allylamino, vinyloxy, vinylaryl and vinylamino group. An aryl group in a vinylaryl group refers to a group derived from an aromatic ring selected from phenyl, naphthyl, thienyl, indolyl, preferably phenyl.

Alternatively, X independently represents a glycidyl ether, a glycidyl imide, or a glycidyl amide group.

n is an integer of 1 to 3, preferably 1.

In a more preferred embodiment, each group A1, A2, A3, A4 and A5, if present, independently represents a fragment (a) as shown above, wherein S/D represents a double bond whereby the double bond is part of an aromatic ring; R represents a group L(X)ₙ, wherein L represents a straight or branched alkylene group having 2 to 5 carbon atoms; X independently represents a polymerizable group selected from a (meth)acryloyloxy group and n is 1.

Preferably, two or three groups selected from A1, A2, A3, A4 and A5 are present in the chemical structure of formula (I). More preferably, two groups selected from A1, A2, A3, A4 and A5 are present.

Depending on the presence or absence of the neighboring groups in formula (I), each group L1 and L2, if present, may be divalent or trivalent. Accordingly, L3, if present, is divalent.

In another embodiment, each group L1, L2 and L3, if present, independently represents a group -O-, -S-, SO₂, or -NR'- (wherein R' represents a hydrogen atom or an alkyl group having 1 to 18 carbon atoms), or a divalent hydrocarbon group having 1 to 8 carbon atoms optionally containing 1 to 5 groups selected from the group of -O-, -NH- and -S- atoms and which may be substituted by 1 to 5 functional groups selected from the group of carboxylic acid groups and hydroxyl groups, when any of L1, L2, and L3 is divalent.

In a preferred embodiment, each group L1, L2 and L3, if present, independently represents a divalent hydrocarbon group, preferably an alkylene goup, having 1 to 5 carbon atoms optionally containing 1 to 3 groups selected from the group of -O-, -NH- and -S- atoms and which may be substituted by 1 to 3 functional groups selected from the group of carboxylic acid groups and hydroxyl groups, when any of L1, L2, and L3 is divalent.

In a more preferred embodiment, each group L1, L2 and L3, if present, independently represents a divalent hydrocarbon group having 1 to 5 carbon atoms which may be substituted by 1 to 3 functional groups selected from the group of carboxylic acid groups and hydroxyl groups, when any of L1, L2, and L3 is divalent. A specific example of L1, L2 and L3 is CH, which forms part of an aromatic ring.

In yet another embodiment, each group L1 and L2, if present, independently represents a group >N- or a trivalent hydrocarbon group having 1 to 5 carbon atoms optionally containing 1 to 3 heteroatoms selected from the group of oxygen, nitrogen and sulfur atoms and which may be substituted by 1 to 3 functional groups selected from the group of carboxylic acid groups and hydroxyl groups when any of L1 and L2 is trivalent.

In a preferred embodiment, each group L1 and L2, if present, independently represents a trivalent hydrocarbon group having 1 to 5 carbon atoms optionally containing 1 to 3 heteroatoms selected from the group of oxygen, nitrogen and sulfur atoms and which may be substituted by 1 to 3 functional groups selected from the group of carboxylic acid groups and hydroxyl groups when any of L1 and L2 is trivalent.

In a more preferred embodiment, each group L1 and L2, if present, independently represents a trivalent hydrocarbon group having 1 to 5 carbon atoms which may be substituted by 1 to 3 functional groups selected from the group of carboxylic acid groups and hydroxyl groups when any of L1 and L2 is trivalent.

In another preferred embodiment, A1, A3, L1 and L2 jointly represent the following formula (II): 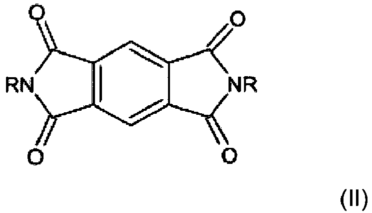 and A2, A4, A5 and L3 are absent.

In yet another preferred embodiment, A1, L1, L3 and A3 jointly represent the following formula (III): 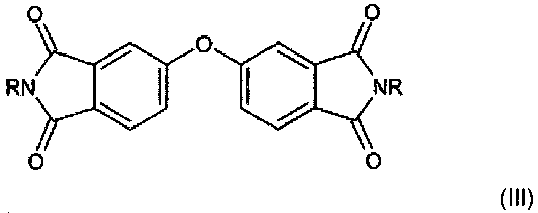 and A2, A4, A5 and L2 are absent.

For a more convenient and practicable application of the dental composition to a patient, the compound of formula (I) which is contained in the composition of the present invention is preferably liquid at 25 °C.

The dental composition is preferably in the form of a highly filled dental composite, a flowable dental composite, pit and fissure sealant, a dental adhesive, a dental cement, root canal sealer, and glass ionomer cement.

The present invention further provides a process for the preparation of a composition comprising a compound of formula (I), which comprises reacting a compound of the following formula (X)

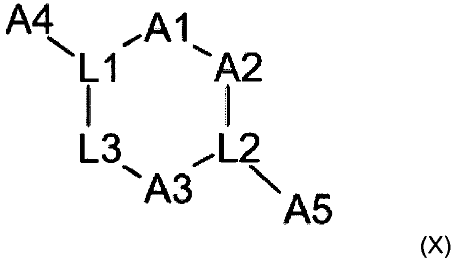

In formula (X), A1, A2, A3, A4, and A5 independently represents a group, which is either present or absent, provided that at least two groups selected from A1, A2, A3, A4, and A5 are present, each group A1, A2, A3, A4, and A5, when present, independently representing a moiety comprising a linear, branched or cyclic hydrocarbon fragment having 0 to 18 carbon atoms and optionally 1 to 5 groups selected from the group of -O-, -NH- and -S-atoms and which may be substituted by 1 to 5 functional groups selected from the group of carboxylic acid groups and hydroxyl groups. In formula (X) each A1, A2, A3, A4, and A5 independently further contains a fragment selected from the following fragments (d): 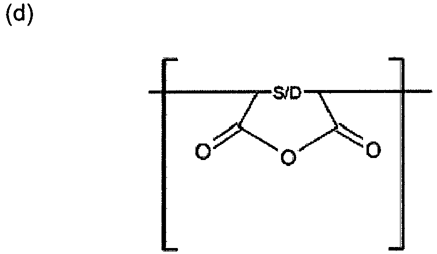 wherein S/D represents a single bond or a double bond whereby the double bond may be part of an aromatic ring.

In formula (X), L1, L2, and L3 are either present or absent, provided that at least one group selected from L1, L2, and L3 is present, and if present, independently represents when any of L1, L2, and L3 is divalent:
a single bond, a group -O-, -S-, SO₂, or -NR'- (wherein R' represents a hydrogen atom or an alkyl group having 1 to 18 carbon atoms), or a divalent hydrocarbon group having 1 to 8 carbon atoms optionally containing 1 to 5 groups selected from the group of -O-, -NH- and -S- atoms and which may be substituted by 1 to 5 functional groups selected from the group of carboxylic acid groups and hydroxyl groups; and
when any of L1 and L2 is trivalent:
   a group >N- or a trivalent hydrocarbon group having 1 to 8 carbon atoms optionally containing 1 to 5 heteroatoms selected from the group of oxygen, nitrogen and sulfur atoms and which may be substituted by 1 to 5 functional groups selected from the group of carboxylic acid groups and hydroxyl groups.

A compound of formula (X) is reacted with a compound of the following formula (XI)

H₂N-L(Y')ₙ (XI)

wherein L represents a linear, branched or cyclic (n+1) valent hydrocarbon group having 2 to 18 carbon atoms which may contain 1 to 5 heteroatoms selected from the group of oxygen, nitrogen and sulfur atoms, and which may be substituted by 1 to 5 functional groups selected from the group of carboxylic acid groups, and hydroxyl groups; Y' represents a hydroxyl group or an amino group; and n is an integer of 1 to 3.

Alternatively, a compound of formula (X) is reacted with a compound of the following formula (XII) 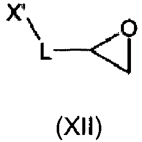 wherein
L methylene group; and
X' is an amino group.

Subsequent to the reaction with a compound of formula (XI), reacting the reaction product with one or more compounds of formula (XII) wherein X' is a leaving group, preferably a halogen atom, or with (meth)acrylic anhydrides for obtaining a compound of formula (I),

The process of the present inventon further comprises combining the compound of formula (I) with a solvent and/or a particulate filler.

Next, the preferred embodiments of a compound represented by formula (X) will be further described.

In one preferred embodiment, S/D in fragment (d) represents a double bond whereby the double bond is part of an aromatic ring.

In another preferred embodiment, each group A1, A2, A3, A4 and A5, if present, independently represents a moiety comprising a linear, branched or cyclic hydrocarbon fragment having 0 to 3 carbon atoms and optionally 1 to 3 groups selected from the group of -O-, -NH- and -S- atoms, and a fragment (d) as shown above, wherein S/D represents a double bond whereby the double bond is part of an aromatic ring.

Preferably, one to three groups selected from A1, A2, A3, A4 and A5 are present in the chemical structure of formula (X). More preferably, two groups selected from A1, A2, A3, A4 and A5 are present.

Depending on the presence or absence of the neighboring groups in formula (X), each group L1 and L2, if present, may be monovalent, divalent or trivalent. Accordingly, L3, if present, may be monovalent or divalent.

In one embodiment, each group L1, L2 and L3, if present, independently represents a hydrogen atom when any of L1, L2, and L3 is monovalent.

In another embodiment, each group L1, L2 and L3, if present, independently represents a group -O-, -S-, SO₂, or -NR'- (wherein R' represents a hydrogen atom or an alkyl group having 1 to 18 carbon atoms), or a divalent hydrocarbon group having 1 to 8 carbon atoms optionally containing 1 to 5 groups selected from the group of -O-, -NH- and -S- atoms and which may be substituted by 1 to 5 functional groups selected from the group of carboxylic acid groups and hydroxyl groups, when any of L1, L2, and L3 is divalent.

In a preferred embodiment, each group L1, L2 and L3, if present, independently represents a divalent hydrocarbon group having 1 to 5 carbon atoms optionally containing 1 to 3 groups selected from the group of -O-, -NH- and -S- atoms and which may be substituted by 1 to 3 functional groups selected from the group of carboxylic acid groups and hydroxyl groups, when any of L1, L2, and L3 is divalent.

In a more preferred embodiment, each group L1, L2 and L3, if present, independently represents a divalent hydrocarbon group having 1 to 5 carbon atoms which may be substituted by 1 to 3 functional groups selected from the group of carboxylic acid groups and hydroxyl groups, when any of L1, L2, and L3 is divalent.

In yet another embodiment, each group L1 and L2, if present, independently represents a group >N- or a trivalent hydrocarbon group having 1 to 5 carbon atoms optionally containing 1 to 3 heteroatoms selected from the group of oxygen, nitrogen and sulfur atoms and which may be substituted by 1 to 3 functional groups selected from the group of carboxylic acid groups and hydroxyl groups when any of L1 and L2 is trivalent.

In a preferred embodiment, each group L1 and L2, if present, independently represents a trivalent hydrocarbon group having 1 to 5 carbon atoms optionally containing 1 to 3 heteroatoms selected from the group of oxygen, nitrogen and sulfur atoms and which may be substituted by 1 to 3 functional groups selected from the group of carboxylic acid groups and hydroxyl groups when any of L1 and L2 is trivalent.

In a more preferred embodiment, each group L1 and L2, if present, independently represents a trivalent hydrocarbon group having 1 to 5 carbon atoms which may be substituted by 1 to 3 functional groups selected from the group of carboxylic acid groups, and hydroxyl groups when any of L1 and L2 is trivalent.

In a preferred embodiment of the compound of formula (XI) shown above, L represents a straight or branched alkylene group having 2 to 18 carbon atoms which may contain 1 to 5 groups selected from the group of oxygen, nitrogen and sulfur atoms, and which may be substituted by 1 to 5 functional groups selected from the group of carboxylic acid groups and hydroxyl groups; and n is an integer of 1 to 3.

In a more preferred embodiment of the compound of formula (XI) shown above, L represents a straight or branched alkylene group having 2 to 5 carbon atoms; and n is 1.

In a preferred embodiment, the compound of formula (X) is pyromellitic dianhydride.

In another preferred embodiment, the compound of formula (X) is 4,4'-oxidiphthalic anhydride.

As the compound of formula (XI), 2-amino ethanol is preferred. In an alternative embodiment, as the compound of formula (XI), 2-amino pentanol is preferred.

The molar ratio of the compound of formula (X) and the compound of formula (XI) in the mixture to be reacted in the first step of the process of the present invention (mol compound of formula (X) /mol compound of formula (XI)) is in the range of from 100:5 to 5:100, preferably in the range from 20:5 to 5:50, more preferably in the range from 2:1 to 5:20.

The reaction conditions of the reaction of the compound of formula (X) and the compound of formula (XI) of the present invention are not particularly limited. Accordingly, it is possible to carry out the reaction in the presence or absence of a solvent. A suitable solvent may be selected from the group of water, dimethyl formamide (DMF), tetrahydrofurane (THF), and dioxane.

The reaction temperature is not particularly limited. Preferably, the reaction is carried out at a temperature of between -10°C to the boiling point of the solvent. Preferably, the reaction temperature is in the range of from 0°C to 80°C.

The reaction time is not particularly limited. Preferably the reaction time is in the range of from 10 minutes to 48 hours, more preferably 1 hour to 36 hours.

The reaction product of the first step of the process of the present invention may be dried by removal of the solvent by reduced pressure. The product may be further purified by washing with a suitable solvent, preferably by washing with ethanol.

The molar ratio of the reaction product from the reaction of compound of formula (X) and the compound of formula (XI) in the first step with the one or more (meth)acrylic anhydrides to be reacted in the second step of the process of the present invention (mol reaction product from first step /mol (meth)acrylic anhydride(s)) is in the range of from 2:1 to 1:100, preferably in the range from 1:2 to 1:50, more preferably in the range from 1:5 to 1:20.

The reaction conditions of the reaction of the reaction product of the first step with the one or more (meth)acrylic anhydrides to be reacted in the second step of the process of the present invention are not particularly limited. Accordingly, it is possible to carry out the reaction in the presence or absence of a solvent.

The reaction temperature is not particularly limited. Preferably, the reaction is carried out in an air-tight container, such as a snap cap utilized vessel, at a temperature of between 50°C and 150°C. More preferably, the reaction is carried out under microwave irritation.

The reaction time is not particularly limited. Preferably, the reaction time is in the range of from 10 minutes to 10 hours, more preferably 15 minutes to 3 hours.

The reaction product of the second step of the process of the present invention may be isolated by precipitation and filtration. The product may be further purified by washing with a suitable solvent. Alternatively, the product of the second step may be purified by dissolving in a suitable organic solvent such as chloroform, followed by extraction with water in order to remove unreacted components.

The present invention provides a dental composition comprising a compound obtainable by the process as defined above. Preferably, such a dental composition has a viscosity of from 1 Pas to 100 Pas at 25°C according to EN ISO 2555:1999.

The dental composition comprising the compound obtainable by the process as defined above is preferably in the form of a highly filled dental composite, a flowable dental composite, pit and fissure sealant, a dental adhesive, a dental cement, root canal sealer, and glass ionomer cement.

The dental composition of the invention may comprise the polymerizable monomer compound of formula (I) or the compound obtained by the process of the present invention in an amount of 5 to 90 wt%, preferably in an amount of 20 to 70 wt% based on the total weight of the composition.

A dental composition according to the present invention comprises the compound as described above and may additionally contain a particulate filler, an initiator system and one or more additional comonomers.

Fillers that are suitable for use in the present invention provide the composite with desired physical and curing properties, such as increased strength, modulus, hardness, reduced thermal expansion, polymerization shrinkage, and stable shelf life. Particulate filler materials may include any kind of filler particles that are suitable for use in dental compositions. The filler to be used in the composition of the present invention may be comprised of inorganic filler materials or organic filler materials or combinations thereof; preferably, the filler is a nanofiller.

Examples of suitable filler particles include, but are not limited to, strontium silicate, strontium borosilicate, strontium aluminosilicate glass, strontium aluminofluorosilicate glass, strontium aluminofluoroborosilicate glass, barium silicate, barium borosilicate, barium fluoroalumino borosilicate glass, barium alumino borosilicate, calcium silicate, calcium alumino silicate glass, calcium alumino fluorosilicate glass, calcium aluminumfluoroborosilicate glass, calcium sodium phosphosilicate, fluoro phosphosilicate, lanthanum aluminosilicate, alumino phosphosilicate, and a combination comprising at least one of these fillers.

Examples of further suitable inorganic filler particles, such as silicon nitrides, titanium dioxide, fumed silica, colloidal silica, submicron silica particles such as pyrogenic silicas, quartz, kaolin ceramics, calcium hydroxy apatite, zirconia, naturally-occurring or synthetic materials, such as glasses derived from, for example Ce, Sb, Sn, Zr, Sr, Ba and Al, feldspar, kaolin, talc, and mixtures thereof.

The filler particles may further comprise organic filler particles, such as filled or unfilled pulverized polycarbonates or polyepoxides. Preferably the surface of these filler particles is treated with a coupling agent in order to enhance the bond between the filler and the matrix. The use of suitable coupling agents includes gamma-methacryloxypropyltrimethoxysilane, gamma-mercaptopropyltriethoxysilane, gamma-aminopropyltrimethoxysilane, and the like.

Further suitable fillers may be selected from fillers currently used in dental restorative compositions.

The filler may have a unimodal or polymodal (e.g., bimodal) particle size distribution. It can also be a crosslinked organic material that is insoluble in the polymerizable compound of the present invention, and is optionally filled with inorganic filler. The filler can be radiopaque, radiolucent or non-radiopaque.

The particulate filler usually has an average particle size of from 0.001 to 100 µm, preferably of from 0.01 to 40 µm as measured, for example, by electron microscopy or by using a conventional laser diffraction particle sizing method as embodied by a MALVERN Mastersizer S or MALVERN Mastersizer 2000 apparatus. The particulate filler may be a multimodal particulate filler representing a mixture of two or more particulate fractions having different average particle sizes. The particulate filler may also be a mixture of particles of different chemical composition. The particulate filler may be surface modified by a surface modifying agent.

The filler may be contained in an amount of from 0.1 to 90 weight%, preferably 40 to 85 weight% based on the total weight of the composition.

The dental composition according to the present invention comprises an initiator system. The initiator system may be based on a redox initiator or on a photoinitiator.

In case the dental composition contains a redox initiator, the amount of reducing agent and oxidizing agent should be sufficient to provide the desired degree of polymerization. Preferably, the mixed but unset cements of the invention contain a combined weight of about 0.01 to about 10%, more preferably about 0.2 to about 5%, and most preferably about 0.3 to about 3% of the reducing agent and oxidizing agent, based on the total weight (including water) of the mixed but unset cement components. The reducing agent or the oxidizing agent can be microencapsulated as described in U.S. Pat. No. 5,154,762. This will generally enhance shelf stability of the cement parts and if necessary permit packaging both the reducing agent and oxidizing agent together. Water-soluble and water-insoluble encapsulants can be employed. Suitable encapsulating materials include cellulosic materials as cellulose acetate, cellulose acetate butyrate, ethyl cellulose, hydroxymethyl cellulose and hydroxyethyl cellulose being preferred. Other encapsulants include polystyrene, copolymers of polystyrene with other vinylic monomers and polymethylmethacrylate, copolymers of methylmethacrylate with other ethylenically-unsaturated monomers. Preferred encapsulants are ethylcellulose and cellulose acetate butyrate. By varying the choice of encapsulant and the encapsulation conditions, the onset of curing can be tailored to start at times ranging from seconds to minutes. The ratio of amount of encapsulant to activator generally ranges from 0.5 to about 10 and preferably from about 2 to about 6.

Suitable oxidizing agents (initiators) include peroxides such as benzoyl peroxide cumene hydroperoxide (CHP), and tert-butyl hydroperoxide, ferric chloride, hydroxylamine (depending upon the choice of reducing agent), perboric acid and its salts, and salts of a permanganate or persulfate anion. Preferred oxidizing agents are peroxides, potassium persulfate, ammonium persulfate and hydrogen peroxide.

Suitable reducing agents (activators) include ascorbic acid, benzyl thiourea ferrous chloride, ferrous sulfate, hydrazine, hydroxylamine (depending upon the choice of oxidizing agent) oxalic acid, thiourea, and salts of a dithionite or sulfite anion. Preferred reducing agents include ascorbic acid and ferrous sulfate.

A photoinitiator should be capable of promoting polymerization of the polymerizable groups on exposure to light of a suitable wavelength and intensity. The photoinitiator preferably is sufficiently shelf-stable and free of undesirable coloration to permit its storage and use under typical dental conditions. Visible light photoinitiators are preferred. Suitable visible light-induced and ultraviolet light-induced initiators include an alpha-diketone (e.g., camphorquinone) with or without additional hydrogen donors (such as sodium benzene sulfinate, amines and amine alcohols). The photoinitiator may be present in an amount sufficient to provide the desired rate of photopolymerization. This amount will be dependent in part on the light source, the thickness of the cement layer to be exposed to radiant energy and the extinction coefficient of the photoinitiator. Preferably, mixed but unset photocurable cements of the invention will contain about 0.01 to about 5%, more preferably from about 0.1 to about 2% photoinitiator, based on the total weight (including water) of the mixed but unset cement components.

The dental composition of the present invention may additionally comprise a polymerization initiator system or compound. As such an initiator, any compound or system which is capable of initiating the polymerization reaction of the composition of the present invention may be suitably used. Preferably, a photoinitiator is used in the composition of the present invention to render the composition photocurable. Herein, a photoinitiator is any system or compound which promotes a polymerization reaction upon exposure to light, e.g. by the release of free radicals.

The polymerization initiator is preferably selected from azobisisobutyronitrile (AIBN), 2,2-azobis(2-amidinopropane)dihydrochloride, 2,2'-azobis(2-methylbutyronitrile), 2,2'-azobis(N,N'-dimethyleneisobutyramidine) dihydrochloride, and 4,4'-azobis(4-cyano pentanoic acid). The initiator may be based on a radical initiator and preferably comprises azobisisobutyronitrile (AIBN). The amount of the polymerization initiator is not particularly limited. Suitably, the amount of the polymerization initiator is in the range of from 0.001 to 5 mol % based on the total amount of the monomers.

Suitable comonomers contain at least one polymerizable functional group. Suitable polymerizable functional groups are ethylenically unsaturated groups (e. g. alkenyl groups and preferably vinyl groups). Preferred examples are substituted and unsubstituted acrylates, methacrylates, or alkenes.

A dental composition according to the present invention may further include a modifying agent for adjusting the curing time of the polymerization reaction.

A dental composition according to the present invention may further contain solvents, pigments, nonvitreous fillers, free radical scavengers, polymerization inhibitors, bisacrylamides such as N,N'-diethyl-1,3-bisacrylamido-propan (BADEP), 1,3-bisacrylamidopropan (BAP), and 1,3-bisacrylamido-2-ethyl-propan (BAPEN), reactive and nonreactive diluents e.g., 2-hydroxyethyl methacrylate (HEMA), hydroxypropyl methacrylate, surfactants (such as to enhance solubility of an inhibitor e. g., polyoxyethylene), coupling agents to enhance reactivity of fillers e.g.,3- (trimethoxysilyl) propyl methacrylate, and rheology modifiers.

Suitable solvents or nonreactive diluents include alcohols such as ethanol and propanol. Suitable reactive diluents are alpha,beta unsaturated monomers for providing altered properties such as toughness, adhesion, and curing time.

Suitable alpha,beta-unsaturated monomers may be acrylates and methacrylates such as methyl acrylate, methyl methacrylate, ethyl acrylate, ethyl methacrylate, propyl acrylate, propyl methacrylate, isopropyl acrylate, isopropyl methacrylate, 2-hydroxyethyl acrylate, 2-hydroxyethyl methacrylate (HEMA), hydroxypropyl acrylate, hydroxypropyl methacrylate, tetrahydrofurfuryl acrylate, tetrahydrofurfuryl methacrylate, glycidyl acrylate, glycidyl methacrylate, the diglycidyl methacrylate of bis-phenol A ("bis-GMA"), glycerol mono- and di- acrylate, glycerol mono- and di- methacrylate, ethyleneglycol diacrylate, ethyleneglycol dimethacrylate, polyethyleneglycol diacrylate (where the number of repeating ethylene oxide units vary from 2 to 30), polyethyleneglycol dimethacrylate (where the number of repeating ethylene oxide units vary from 2 to 30 especially triethylene glycol dimethacrylate ("TEGDMA"), neopentyl glycol diacrylate, neopentylglycol dimethacrylate, trimethylolpropane triacrylate, trimethylol propane trimethacrylate, mono-, di-, tri-, and tetraacrylates and methacrylates of pentaerythritol and dipentaerythritol, 1,3-butanediol diacrylate, 1,3-butanediol dimethacrylate, 1,4-butanedioldiacrylate, 1,4-butanediol dimethacrylate, 1,6-hexane diol diacrylate, 1,6-hexanediol dimethacrylate, di-2-methacryloyloxethyl hexamethylene dicarbamate, di-2-methacryloyloxyethyl trimethylhexanethylene dicarbamate, di-2-methacryloyl oxyethyl dimethylbenzene dicarbamate, methylene-bis-2-methacryloxyethyl-4-cyclohexyl carbamate, di-2-methacryloxyethyl-dimethylcyclohexane dicarbamate, methylene-bis-2-methacryloxyethyl-4-cyclohexyl carbamate, di-1-methyl-2-methacryloxyethyl-trimethyl-hexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethylhexamethylene dicarbamate, di-1-chloromethyl-2-methacryloxyethyltrimethylhexamethylene dicarbamate, di-1-chloromethyl-2-methacryloxyethyldimethylbenzene dicarbamate, di-1-chloromethyl-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyltrimethylhexamethylene dicarbamate, di-1-methyl-2-methacryloxyethyl-dimethylbenzene dicarbamate, di-1-methyl-2-metha-cryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-1-chloromethyl-2-methacryloxyethyl4-cyclohexyl carbamate, 2,2'-bis(4-methacryloxyphenyl)propane, 2,2'bis(4-acryloxyphenyl)propane, 2,2'-bis[4(2-hydroxy-3-methacryloxy-phenyl)]propane, 2,2'-bis[4(2-hydroxy-3-acryloxy-phenyl)propane, 2,2'-bis(4-methacryloxyethoxyphenyl)propane, 2,2'-bis(4-acryloxyethoxyphenyl)propane, 2,2'-bis(4-methacryloxypropoxyphenyl)propane, 2,2'-bis(4-acryloxypropoxyphenyl)propane, 2,2'-bis(4-methacryloxydiethoxyphenyl)propane, 2,2'-bis(4-acryloxydiethoxyphenyl)propane, 2,2'-bis[3(4-phenoxy)-2-hydroxypropane-1-methacrylate]propane,and 2,2'-bis[3(4-phenoxy)-2-hydroxypropane-1-acrylate]propane, may be mentioned. Other suitable examples of polymerizable components are isopropenyl oxazoline, vinyl azalactone, vinyl pyrrolidone, styrene, divinylbenzene, urethane acrylates or methacrylates, epoxy acrylates or methacrylates and polyol acrylates or methacrylates. Mixtures of alpha,beta-unsaturated monomers can be added if desired. Preferably, the mixed but uncured dental compositions of the invention will contain a combined weight of about 0.5 to about 40%, more preferably about 1 to about 30%, and most preferably about 5 to 20% water, solvents, diluents and alpha,beta-unsaturated monomers, based on the total weight (including such water, solvents, diluents and alpha,beta-unsaturated monomers) of the mixed but uncured dental composition components.

An example of a suitable free radical scavenger is 4-methoxyphenol. An example of a suitable inhibitor is hydroxytoluene or butylated hydroxytoluene (BHT). The amount of inhibitor may be selected from 0.001 to 2% and preferably from 0.02 to 0.5% based on the total weight of the copolymer/comonomer/water mixture.

The invention will now be further illustrated by the following Examples.

### Examples

### Example 1

### Bis-[N-hydroxvethyl]-pyromellitic diimide

A mixture of 10 g (46 mmol) pyromellitic dianhydride and 7.3 g (120 mmol) 2-amino ethanol dissolved in 60 ml DMF had been refluxed for 9 hours. From the resulting mixture the solvent had been removed by reduced pressure and the obtained solid was washed throughout with 750 ml of ethanol. Yield: 6.42 g (55 %), Formula: C₁₄H₁₂N₂O₆ (304.25 g/mol)
¹H-NMR [300 MHz, DMSO, ppm]: δ 8.19 (s, 2H, Pos. 3,6),4.88 (t, 2H, Pos. 23, 24), 3.69 (m, 4H, Pos. 17, 21), 3.6 (m, 4H, Pos.18, 22) 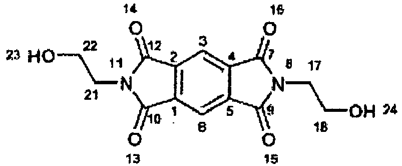 IR [cm⁻¹]: v 3034 (w, Ar-H); 2946 - 2883 (m, -C-H stretching vib.); 1771, 1697 (s, Imide) MS (El): 304 [M]

### Bis-[N-3-methacryloylethyl]-pyromellitic diimide

A snap cap utilized microwave vessel filled with Bis-[N-hydroxyethyl]-pyromellitic diimide and methacrylic anhydride (ratio 1:10) were brought to reaction under microwave irritation (30 Watt, 120°C, 45 min). The resulting beige solid was poured in 1000 ml of water, stirred for 3 hours, filtered and extracted by a mixture of chloroform and water/KOH (1 molar) and dried by magnesia sulfate. The solvent was finally removed by reduced pressure. Yield: 0.43 g (65%), Formula: C₂₂H₂₀N₂O₈ (440.40 g/mol)
¹H-NMR [300 MHz, CDCl₃, ppm]: δ 8.28 (s, 2H, Pos. 3, 6), 6.05 (m, 2H, Pos. 25, 31), 5.56 (m, 2H, Pos. 25, 31), 4.39 (t, 4H, Pos. 18,20),4.06 (t, 4H, Pos. 17, 19), 1.87 - 1.83 (m, 6H, Pos. 26, 32) 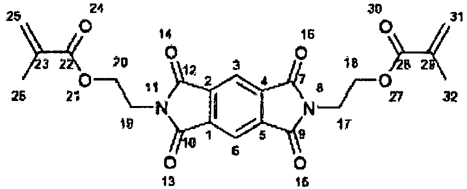 IR [cm⁻¹]: v 3038, (w, Ar-H); 2964 (m, -C-H stretching vib.), 1772, 1701 (s, Imide), 1637 (-C=C-)
MS (El): 440 [M]

### Example 2

### 5,5'-Bis[phthalimido-N-5-hydroxy pentyl]ether

A mixture of 8 g (26 mmol) 4,4'-oxidiphthalic anhydride and 6,6 g (64 mmol) 5-amino pentanol dissolved in 80 ml DMF had been refluxed for 9 hours. From the resulting mixture the solvent had been removed by reduced pressure and the obtained solid was washed throughout with 750 ml of ethanol. Yield: 7.26 g (58 % of th.), Formula: C₂₆H₂₈N₂O₇ (480.51 g/mol), mp: 133°C
¹H-NMR [300 MHz, DMSO, ppm]: δ 7.92 (d, 2H, Pos. 6, 18), 7.55 - 7.50 (m, 4H, Pos. 3, 5, 15, 17), 4.35 (t, 2H, Pos. 29, 35), 3.55 (m, 4H, Pos. 28, 34), 3.38 - 3.33 (m, 4H, Pos. 24, 30), 1.63 -1.53 (m, 4H, Pos. 27, 33), 1.47 -1.38 (m, 4H, Pos. 25, 31), 1.34 -1.25 (m, 4H, Pos. 26, 32). 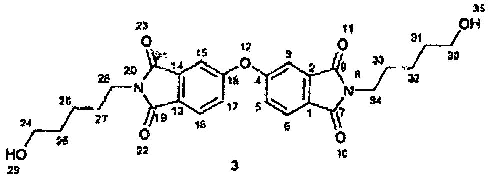 IR [cm⁻¹]: v 3066 (w, Ar-H); 2936 - 2882 (m, -C-H stretching vib.); 1765, 1691 (s, Imide)

### 5,5'-Bis[phthalimido-pentan-1,5 diyl methacryloyl]ether

A snap cap utilized microwave vessel filled with 5,5'-Bis[phthalimido-N-5-hydroxy pentyl]ether and methacrylic anhydride (ratio 1:10) were brought to reaction under microwave irritation (30 Watt, 120°C, 45 min). The resulting beige solid was poured in 1000 ml of water, stirred for 3 hours, filtered and extracted by a mixture of chloroform and water/KOH (1 molar) and dried by magnesia sulfate. The solvent was removed by reduced pressure. Yield: 5.5 g (53%), Formula: C₃₄H₃₆N₂O₉ (616.66 g/mol)
¹H-NMR [300 MHz, CDCl₃, ppm]: δ 7.85 (d, 2H, Pos. 6, 18), 7.41 - 7.32 (m, 4H, Pos. 3, 5, 15, 17), 6.07-6.02 (m, 2H, Pos. 39, 45), 5.54 - 5.47 (m, 2H, Pos. 39, 45), 4.11 (t, 4H, Pos. 24,30),3.67 (t, 4H, Pos. 28, 34), 1.91 -1.88 (m, 6H, Pos. 44, 40), 1.70 (m, 8H, Pos. 25, 27, 31, 33), 1.46 - 1.38 (m, 4H, Pos. 26, 32). 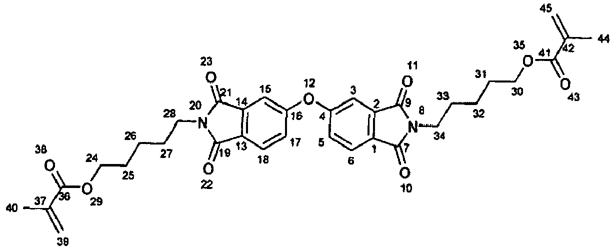 IR [cm⁻¹]: v 3040 (w, Ar-H); 2942 - 2863 (m, -C-H stretching vib.); 1768, 1701 (s, Imide), 1637 (m)
MS (Maldi-TOF): 617.2 [M]

### Example 3

### 5,5'-Bis[phthalimido-N-2-hydroxy ethyl]ether

A mixture of 5g (16 mmol) 4,4'-oxidiphthalic anhydride and 2,46 g (40,3 mmol) 2-amino ethanol dissolved in 40 ml DMF had been refluxed for 9 hours. From the resulting mixture the solvent had been removed by reduced pressure and the obtained solid was washed throughout with 750 ml of ethanol. Yield: 2.9 g (45 %), Formula: C₂₀H₁₆N₂O₇ (396.35 g/mol), mp: 169 °C
¹H-NMR [300 MHz, DMSO, ppm]: δ 7.96 - 7.91 (m, 2H, Pos. 6, 18), 7.55 - 7.49 (m, 4H, Pos. 3, 5, 15, 17), 4.83 (t, 2H, Pos. 32, 35), 3.66 - 3.54 (m, 8H, Pos. 27, 28, 33, 34) 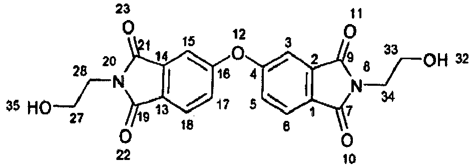 IR [cm⁻¹]: v 3072 (w, Ar-H); 2946 (m, -C-H stretching vib.); 1760, 1690 (s, Imide)

### 5,5'-Bis[phthalimido-ethan-1,2 diyl methacryloyl]ether

A snap cap utilized microwave vessel filled with 5,5'-Bis[phthalimido-N-2-hydroxy ethyl]ether and methacrylic anhydride (ratio 1:10) were brought to reaction under microwave irritation (30 Watt, 120°C, 45 min). The resulting beige solid was poured in 1000 ml of water, stirred for 3 hours, filtered and extracted by a mixture of chloroform and water/KOH (1 molar) and dried by magnesia sulfate. The solvent was removed by reduced pressure. Yield: 10.3 g (51 %), Formula: C₂₈H₂₄N₂O₉ (532.51g/mol), mp: 125.55 °C (DSC)
¹H-NMR [300 MHz, CDCl₃, ppm]: δ 7.86 (m, 2H, Pos. 6, 18), 7.44 - 7.32 (m, 4H, Pos. 3, 5, 15, 17),6.06 - 6.01 (m, 2H, Pos. 39, 44), 5.55 - 5.49 (m, 2H, Pos. 39, 44), 4.33 (m, 4H, Pos. 27,33),3.97 (m, 4H, Pos. 28, 34) 1.87-1.83 (m, 6H, Pos. 38, 43) 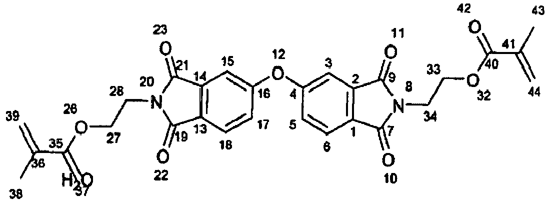 IR [cm⁻¹]: v 3036 (w, Ar-H); 2953, 2926 (m, -C-H stretching vib.); 1774, 1706 (s, Imide) 1632 (m, -C=C-)

### Example 4

### Bis-[N-glycidyl]-pyromellitic-diimide

0.8g Pyromellitic diimide (3.703mmol) and 0.25g tetrabutylammonium hydrogen sulfate (0.74mmol) were given into a three-necked flask equipped with a septum, condenser and thermometer. Under ice cooling, a 60-fold excess of epichlorohydrin (17.42ml) was added slowly to the educts. After that, the mixture has been heated up to 110°C for 24 hours. Approximately one hour at temperature, the educts were dissolved by epichlorohydrin and the formerly colorless solution turned orange. A white solid product was obtained by precipitation with cold ethanol which is washed several times with ethanol to remove remaining epichlorohydrin. 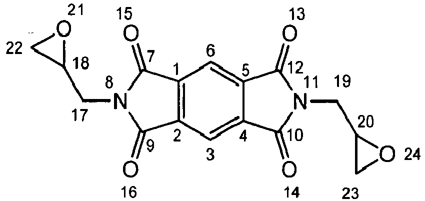 Yield: 0.78 g (69.55 %) Formula: C₁₆H₁₂N₂O₆ (328.25 g/mol)
Elemental analysis, calc.: C 58.54%, H 3.68%, N 8.53% found: C 57.29%, H 3.58%, N 8.42%)
¹H-NMR [ppm]: (300 MHz, DMSO) δ 8.24 (s, 2H, Pos. 3, 6), δ 3.82 (m, 4H, Pos. 17, 19), δ 3.23 (m, 2H, Pos. 18, 20), δ 2.76 (m, 2H, Pos. 22a, 23a), δ 2.62 (m, 2H, Pos. 22b, 23b)
¹³C-NMR [ppm]: (300 MHz, DMSO) δ 166.07 (Pos. 7, 9, 10, 12); δ 136.93 (Pos. 1, 2, 4, 5); δ 117.49 (Pos. 3, 6); δ 48.71 (Pos. 18, 20); δ 45.05 (Pos. 22, 23); δ 39.68 (Pos. 17, 19)
IR (cm⁻¹): □ 1781, 1697 (s, Imide); 1258, 899, 793 (m, epoxid-ring) cm⁻¹
GC/MS: 328 [M]
Mp: ∼190°C

### Example 5

### Bis-[N-Diglycidyl]-3,3',4,4'-Diphenylsulfontetracarboxylic-diimide

3.5 g of 3,3',4,4'-diphenylsulfontetracarboxylicdiimide (9.82 mmol) and 0.71 g tetrabutylammonium hydrogen sulfate (1.79 mmol) were placed into a three-necked flask equipped with a septum, condenser and thermometer. Under ice cooling, a 50-fold excess of epichlorohydrin (40 ml) was added slowly to the educts. After that, the mixture has been heated up to 110°C for 24 hours. Approximately one hour at temperature, the educts were dissolved by epichlorohydrin and the formerly colorless solution turned dark brown. The product precipitated after cooling to room temperature, sucked off from solvent and has been washed several times with ethanol to remove remaining epichlorohydrinfor providing a white solid. 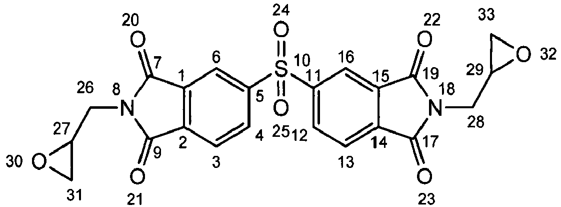 Yield: 3.2 g (69%) Formula: C₂₂H₁₆N₂O₈S (468.44 g/mol)
Elemental analysis, calc.: C 56.41%, H 3.44%, N 5.98% found: C 55.23%, H 3.46%, N 5,78%
¹H-NMR [ppm]: (300 MHz, DMSO) δ 8.56 - 8.54 (m, 4H, Pos. 4, 12, 6, 16), δ 8.12 - 8.10 (m, 2H, Pos. 3, 13), δ 3.78 (m, 4H, Pos. 26, 28), δ 3.18 (m, 2H, Pos. 27, 29), δ 2.71 (m, 2H, Pos. 31 a, 33a),δ 2.57 (m, 2H, Pos. 31b, 33b)
¹³C-NMR [ppm]: (300 MHz, DMSO) δ 166.22 (Pos. 7, 9, 17, 19); δ 145.13 (Pos. 5, 11); δ 136.14 (Pos. 2, 14); δ 134.19 (Pos. 1, 15); δ 133.07 (Pos. 4, 12); δ 124.64 (Pos. 3, 13); δ 122.52 (Pos. 6, 16); δ 48.68 (Pos. 27, 29); δ 44.93 (Pos. 31, 33); δ 39.43 (Pos. 26, 28)
IR: v 1772, 1707 (s, Imide); 1258, 906, 761 (m, epoxid-ring) cm⁻¹
mp: ∼ 220°C

### Example 6

### Bis-[N-Diglycidyl]-4,4'Oxydiphthalic -diimide

2 g 4,4' Oxydiphthalic diimide (6.48 mmol) and 0,44 g tetrabutylammonium hydrogen sulfate (1.3 mmol) were placed into a three-necked flask equipped with a septum, condenser and thermometer. Under ice cooling, a 60-fold excess of epichlorohydrin (30 ml) was added slowly to the educts. Subsequently, the mixture was heated to 110°C for 24 hours. Approximately one hour at temperature, the educts were dissolved by epichlorohydrin and a yellow colored solution was obtained. The solution was cooled to room temperature and washed twice with 100 ml H₂O. The organic layer was dried with MgSO₄ and distilled to one third of its initial volume. The product was precipitated with ethyl ether (-18°C) and dried under constant vacuum. 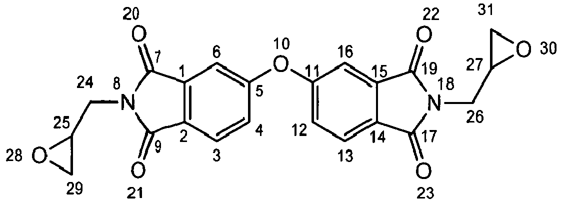 Formula: C₂₂H₁₆N₂O₇ (480.51 g/mol)
Elemental analysis, calc.: C 62.86%, H 3.84%, N 6.66%) found: C 59.68%, H 4.46%, N 6.34%)
¹H-NMR [ppm]: (300 MHz, DMSO) δ 7.98 -7.94 (m, 2H, Pos. 3, 13), δ 7.57 - 7.53 (m, 4H, Pos. 4, 6, 12, 16), δ 3.77 (m, 4H, Pos. 24, 26), δ 3.19 (m, 2H, Pos. 25, 27), δ 2.74 (m, 2H, Pos. 29a, 31 a), δ 2.57 (m, 2H, Pos. 29b, 31 b)
¹³C-NMR [ppm]: (300 MHz, DMSO) δ 166.87 - 166.7 (Pos. 7, 9, 17, 19); δ 160.61 (Pos. 5, 11); δ 134.35 (Pos. 1, 15); δ 127.05 (Pos. 3, 13); δ 125.7 (Pos. 2, 14); δ 124.55 (Pos. 4, 12); δ 113.62 (Pos. 6, 16); δ 48.75 (Pos. 25, 27); δ 45.00 (Pos. 29, 31); δ 39.16 (Pos. 24, 26)
mp: 55-60°C

### Example 7

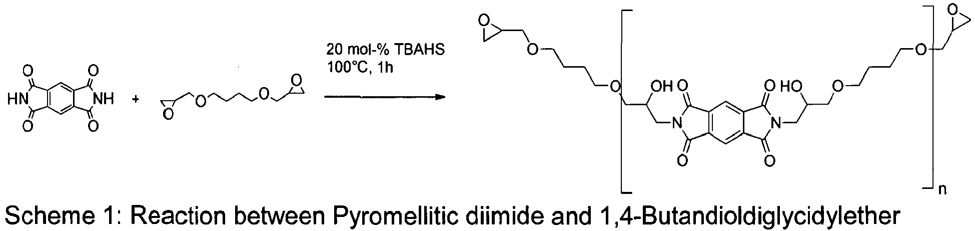

2.16 g Pyromellitic diimide (10 mmol), 0.68 g Tetrabutylammonium hydrogensulfate (2 mmol) and 100 mmol 1,4-Butandioldiglycidylether (20 g) were given into a one-neck flask equipped with a reflux condenser. The white suspension has been heated up to 100°C under high stirring. After approx. 1 hour the suspension was dissolved and cooled down to room temperature immediately. After that, the crude product was precipitated in cold diethyl ether. The obtained white product was dried under constant vacuum.
Yield : 5.4 g
mp (DSC) : :73°C-85°C

### Example 8

### Heck reaction of 4-Bromophthalic imide with 1,7-Octadien (Heck condition)

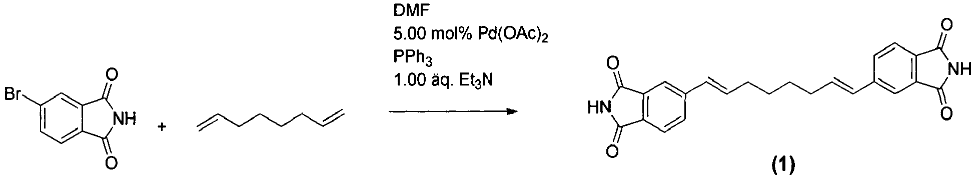

1 g 4-Bromophthalic imide (6.167 mmol), a two-fold excess 1,7-Octadien (12.33 mmol) and the catalysator system were solved in 50 ml DMF. The catalysator system consisted of Palladiumacetate (5 mol-% to Imide: 0.31 mmol), 0.92 mmol Triphenylphosphane and 6.17 mmol Triethylamin. The solution has been heated for 24 h at 150 °C (Control of reaction progress via thin layer chromatography with CH₂Cl₂: n-Hexane [5:1]). From the resulting dark brown solution the solvent was removed by reduced pressure. The obtained viscous brown raw product was solved with 80 ml CH₂Cl₂ and washed three times (3 x 200 ml) with a saturated H₂O/NaCl solution. After that the CH₂Cl₂-phase was dried with MgSO₄ and the solvent was removed by reduced pressure. The viscous brown product (1) was dried under constant vacuum. 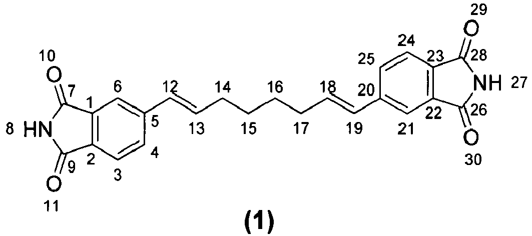 Yield : quant.
IR : v 1772, 1717 (s, Imide) cm⁻¹
MS / ESI : 401.14972 m/z (M+H⁺)

### Epoxidation of (1)

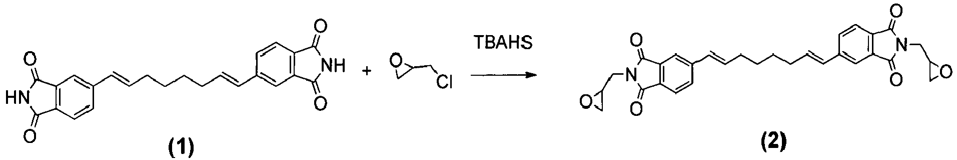

0.3 g of Heck product (1) (0.75 mmol) and 0.05 g Tetrabutylammonium hydrogen sulfate (0.149 mmol) were given into a three neck flask equipped with a septum, condenser and thermometer. Under ice cooling, a 60-fold excess of Epichlorohydrin (3.51 ml) was added slowly to the educts. The educts were soluted rapidly after addition of Epichlorohydrin. The brown, clear solution was heated up to 110°C for 24h. After that, remaining Epichlorohydrin was removed via vacuum distillation. The obtained brown raw product was solved with 30 ml CHCl₃ and washed two times (2 x 30 ml) with water. After that the CHCl₃-phase was dried with MgSO₄ and the solvent was removed by reduced pressure. The low-viscous brown product (2) was dried under constant vacuum.

### Example 9

### Michael addition of Pyromellitic diimide with cross linking agent

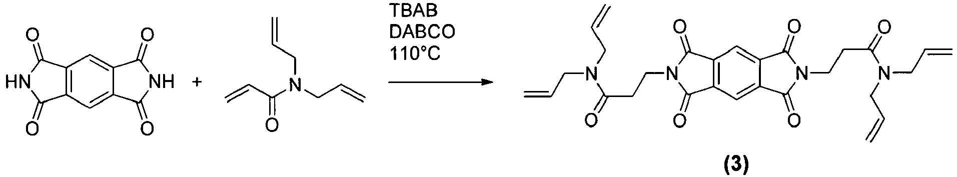

0.15 g Pyromellitic diimide (0.69 mmol), 0.44 g Tetrabutylammonium bromid (1.39 mmol) and 0.155 g DABCO (1.38 mmol) were given into a one-neck flask equipped with a reflux condenser. The mixture has been heated to 110°C under high stirring. Molten TBAB (m.p. 102 °C) creates a homogeneous reaction media and is assumed to behave as an ionic liquid".^{[1]} After that, the cross linking agent (0.315 g; 2.08 mmol) was added to the reaction medium and heated for 18 h. The bright brown raw product was cooled to room temperature. Then chloroform (20 ml) was added and the resulting mixture was washed with water (3 x 100 ml). The chloroform was removed and the obtained brown product (3) was dried under constant vacuum.
Yield : quant.
MS / ESI : 519.22379 m/z (M+H⁺)

## Claims

1. A dental composition comprising a compound of the following formula (I) 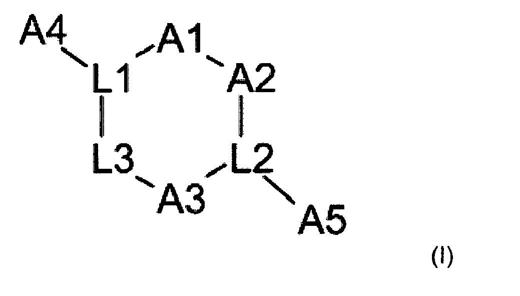 wherein
A1, A2, A3, A4, and A5 independently represents a group, which is either present or absent, provided that at least two groups selected from A1, A2, A3, A4, and A5 are present, each group A1, A2, A3, A4, and A5, when present, independently representing a moiety comprising
○ a linear, branched or cyclic hydrocarbon fragment having 0 to 18 carbon atoms and optionally 1 to 5 groups selected from the group of -O-, -NH- and -S- atoms and which may be substituted by 1 to 5 functional groups selected from the group of carboxylic acid groups and hydroxyl groups; and
○ a fragment selected from the following fragments (a) to (c):
(a) 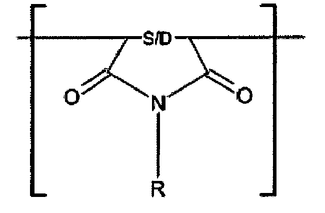
(b) 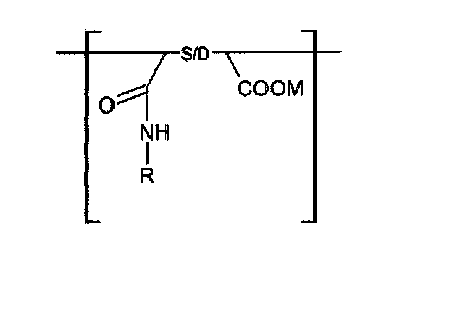
(c) 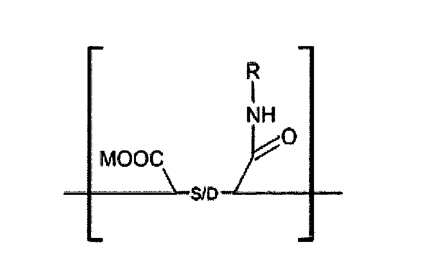 wherein
M represents a hydrogen atom or a monovalent cation;
S/D represents a single bond or a double bond whereby the double bond may be part of an aromatic ring;
R represents a groups L(X)ₙ,
wherein
L represents a single bond or a linear, branched or cyclic (n+1) valent hydrocarbon group having 2 to 18 carbon atoms which may contain 1 to 5 heteroatoms selected from the group of oxygen, nitrogen and sulfur atoms, and which may be substituted by 1 to 5 functional groups selected from the group of carboxylic acid groups, and hydroxyl groups;
X independently represents either
○ a polymerizable group selected from a (meth)acryloyloxy group, an allyl oxy group, allylamino, vinyloxy, vinylaryl and vinylamino group, or
○ a glycidyl ether, a glycidyl amine, or a glycidyl amide group, whereby in case L is a single bond, a glycidyl moiety forms a glycidyl imide in a fragment of formula (a) and a glycidyl amide in a fragment of any one of formula (b) and (c); and
n is an integer of 1 to 3;
L1, L2, and L3 are either present or absent, provided that at least one group selected from L1, L2, and L3 is present, and if present, independently represents
• when any of L1, L2, and L3 is divalent:
a single bond, a group -O-, -S-, SO₂, or -NR'- (wherein R' represents a hydrogen atom or an alkyl group having 1 to 18 carbon atoms), or a divalent hydrocarbon group having 1 to 8 carbon atoms optionally containing 1 to 5 groups selected from the group of -O-, -NH- and -S- atoms and which may be substituted by 1 to 5 functional groups selected from the group of carboxylic acid groups and hydroxyl groups; and
• when any of L1 and L2 is trivalent:
a group >N- or a trivalent hydrocarbon group having 1 to 8 carbon atoms optionally containing 1 to 5 heteroatoms selected from the group of oxygen, nitrogen and sulfur atoms and which may be substituted by 1 to 5 functional groups selected from the group of carboxylic acid groups and hydroxyl groups,
said dental composition further comprising a solvent and/or a particulate filler.

2. The dental composition according to claim 1, wherein two groups selected from A1, A2, A3, A4, and A5 are present.

3. The dental composition according to claim 1 or 2, wherein A1, A3, L1 and L2 jointly represent the following formula (II): 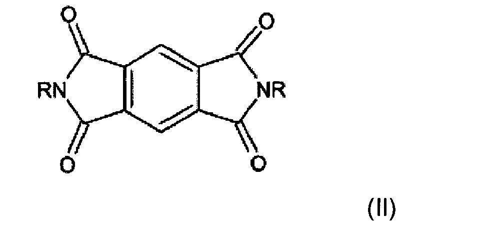 and A2, A4, A5 and L3 are absent.

4. The dental composition according to claim 1 or 2, wherein A1, L1, L3 and A3 jointly represent the following formula (III): 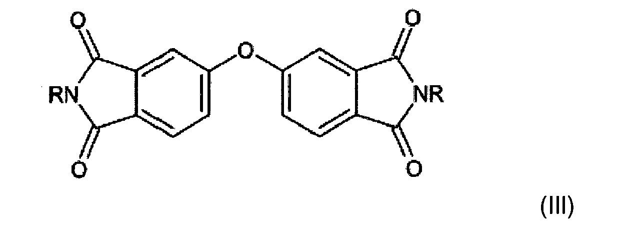 and A2, A4, A5 and L2 are absent.

5. The dental composition according to any one of the preceding claims, wherein R represents a groups L(X)ₙ, wherein
L represents a straight or branched alkylene group having 2 to 18 carbon atoms which may contain 1 to 5 groups selected from the group of -O-, -NH- and -S- atoms, and which may be substituted by 1 to 5 functional groups selected from the group of carboxylic acid groups and hydroxyl groups;
X a polymerizable group independently selected from a (meth)acryloyloxy group;
n is an integer of 1 to 3;

6. The dental composition according to any one of the preceding claims, wherein n is 1.

7. The dental composition according to any one of the preceding claims, wherein the compound of formula (I) is liquid at 25 °C, or soluble at 25 °C in at least 1.0 % wt/v, more preferably, at least 5 % wt/v in a reactive diluent selected from the group consisting of ethylene glycol dimethacrylate, diethylene glycol dimethacrylate, triethylene glycol dimethacrylate, glycerine dimethacrylate, trimethylolpropane tri(meth)acrylate and 2-hydroxyethyl methacrylate.

8. The dental composition according to any one of the preceding claims, which is a highly filled dental composite, a flowable dental composite, pit and fissure sealant, a dental adhesive, a dental cement, root canal sealer, and glass ionomer cement.

9. A process for the preparation of a dental composition comprising a compound of formula (I), which comprises
(i) reacting a compound of the following formula (X) 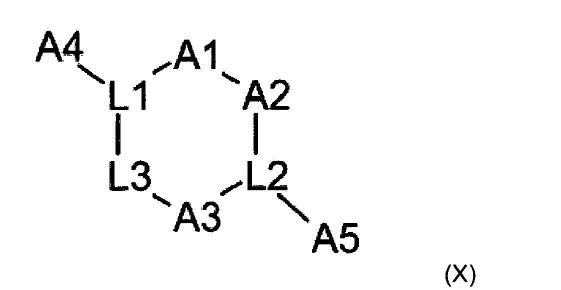 wherein
A1, A2, A3, A4, and A5 independently represents a group, which is either present or absent, provided that at least two groups selected from A1, A2, A3, A4, and A5 are present, each group A1, A2, A3, A4, and A5, when present, independently representing a moiety comprising
○ a linear, branched or cyclic hydrocarbon fragment having 0 to 18 carbon atoms and optionally 1 to 5 groups selected from the group of -O-, -NH- and -S- atoms and which may be substituted by 1 to 5 functional groups selected from the group of carboxylic acid groups and hydroxyl groups; and
○ a fragment selected from the following fragments (d):
(d) 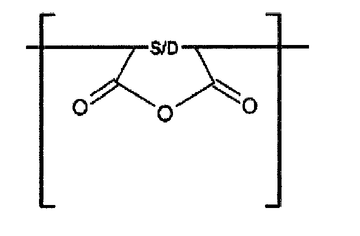 wherein
S/D represents a single bond or a double bond whereby the double bond may be part of an aromatic ring;
L1, L2, and L3 are either present or absent, provided that at least one group selected from L1, L2, and L3 is present, and if present, independently represents
○ when any of L1, L2, and L3 is divalent:
a single bond, a group -O-, -S-, SO₂, or -NR'- (wherein R' represents a hydrogen atom or an alkyl group having 1 to 18 carbon atoms), or a divalent hydrocarbon group having 1 to 8 carbon atoms optionally containing 1 to 5 groups selected from the group of -O-, - NH- and -S- atoms and which may be substituted by 1 to 5 functional groups selected from the group of carboxylic acid groups and hydroxyl groups; and
○ when any of L1 and L2 is trivalent:
a group >N- or a trivalent hydrocarbon group having 1 to 8 carbon atoms optionally containing 1 to 5 heteroatoms selected from the group of oxygen, nitrogen and sulfur atoms and which may be substituted by 1 to 5 functional groups selected from the group of carboxylic acid groups and hydroxyl groups with a compound of the following formula (XI)
H₂N-L(Y')ₙ (XI)
wherein
L represents a linear, branched or cyclic (n+1) valent hydrocarbon group having 2 to 18 carbon atoms which may contain 1 to 5 heteroatoms selected from the group of oxygen, nitrogen and sulfur atoms, and which may be substituted by 1 to 5 functional groups selected from the group of carboxylic acid groups, and hydroxyl groups;
Y' represents a hydroxyl group or an amino group; and
n is an integer of 1 to 3; or
with a compound of the following formula (XII) 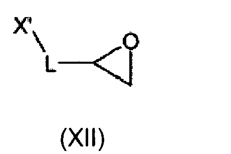 wherein
L methylene group; and
X' is an amino group; and
(ii) subsequent to the reaction with a compound of formula (XI), reacting the reaction product with one or more compounds of formula (XII) wherein X' is a leaving group, preferably a halogen atom, or with (meth)acrylic anhydrides for obtaining a compound of formula (I), and further comprising
(iii) combining the compound of formula (I) with a solvent and/or a particulate filler.

10. A dental composition comprising a compound obtainable according to the process according to claim 9.

11. The dental composition according to claim 10, wherein the composition obtainable according to claim 10 has a viscosity of from 1 Pas to 100 Pas at 25 °C according to EN ISO 2555:1999.

12. The dental composition according to any one of claims 10 and 11, which is a highly filled dental composite, a flowable dental composite, pit and fissure sealant, a dental adhesive, a dental cement, root canal sealer, and glass ionomer cement.

13. Use of a compound obtainable according to the process of claim 9 for the preparation of a dental composition.
